# EUROPÄISCHE PATENTSCHRIFT

(11) **EP 1 745 762 B1**
(45) Veröffentlichungstag und Bekanntmachung des Hinweises auf die Patenterteilung: **16.08.2017**
(21) Anmeldenummer: 06014088.6
(22) Anmeldetag: 07.07.2006
(51) Int. Cl.: A61F 2/18

(54) **Gehörknöchelchenprothese**
Auditory ossicles prosthesis
Prothèse d'osselets de l'oreille

(30) Priorität: 21.07.2005 DE 202005011485 U; 11.02.2006 DE 202006002196 U
(43) Veröffentlichungstag der Anmeldung: 24.01.2007
(73) Patentinhaber: Heinz Kurz GmbH Medizintechnik, 72144 Dusslingen (DE)
(72) Erfinder: Wengen, Dr Daniel Felix, 4102 Binningen (CH); Steinhardt, Uwe, 72145 Hirrlingen (DE)
(74) Vertreter: Kohler Schmid Möbus Patentanwälte

(56) Entgegenhaltungen:
- EP-A1- 1 181 907
- EP-A2- 1 073 313
- DE-U1- 29 609 687
- DE-U1-202004 001 008
- DE-U1-202005 011 485
- US-A1- 2003 130 734
- US-A1- 2004 064 183

## Beschreibung

Die Erfindung betrifft eine Gehörknöchelchenprothese, die mindestens ein Glied der menschlichen Gehörknöchelchenkette ersetzt oder überbrückt, wobei die Gehörknöchelchenprothese zumindest an einem Ende ein als erster elastischer Clip ausgebildetes Befestigungselement zur mechanischen Verbindung mit einem Glied der Gehörknöchelchen-Kette aufweist, welches in Form einer einseitig nach außen hin offenen Klammer mit einer Außenöffnung zur Aufnahme dieses Gliedes, mit dem die mechanische Verbindung hergestellt werden soll, gestaltet ist, und wobei die Klammer nach der Implantation der Prothese dieses Glied mit zwei Bereichen, die an ihren der Außenöffnung gegenüber liegenden Enden über einen mit Abstand von diesem Glied verlaufenden Abschnitt miteinander verbunden sind, formschlüssig umgreift.

Eine solche den Steigbügel überbrückende Prothese ist im deutschen Gebrauchsmuster DE 202 12 771 U1 der Anmelderin bereits beschrieben. Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Ambossfortsatz der menschlichen Gehörknöchelchenkette befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Eine aus dem deutschen Gebrauchsmuster DE 296 09 687 U1 der Anmelderin bekannte Gehörknöchelchenprothese, die ein "Vorgänger"-Modell der in der DE 202 12 771 U1 beschriebenen Prothese darstellt, zeichnet sich dadurch aus, dass sie sich durch einfaches Aufklippen auf den langen Ambossfortsatz relativ leicht implantieren lässt. Sie hält allein durch die Klemmwirkung des Clips. Weitere Befestigungsmittel sind überflüssig.

Dennoch könnte es im Verlauf einer Implantation zu Verkippungen der Prothese kommen, wodurch die Operation für den Chirurgen erschwert würde. Um diesem Problem zu begegnen, ist bei der aus der eingangs zitierten DE 202 12 771 U1 bekannten Gehörknöchelchenprothese die Klammer mit mindestens einem ihrer Schenkel verlängert, der über die Öffnung nach außen in Form eines Bogens ragt, mit dem die Prothese am langen Ambossfortsatz des menschlichen Mittelohrs vor dem Aufschieben aufhängbar ist. Durch dieses Aufhängen der Prothese am langen Ambossfortsatz gewinnt der Operateur die Möglichkeit, das Operationsbesteck zu wechseln und z. B. ein Häkchen zum Aufschieben der Prothese auf den langen Ambossfortsatz zu verwenden.

Der vorliegenden Erfindung liegt demgegenüber nun die Aufgabe zugrunde, die aus der DE 202 12 771 U1 bekannte Steigbügelprothese mit möglichst einfachen Mitteln so auszugestalten, dass ihre Implantation im Mittelohr noch weiter vereinfacht wird.

Diese Aufgabe wird mit einer Gehörknöchelchenprothese der eingangs genannten Art erfindungsgemäß dadurch gelöst, dass der die beiden Bereiche verbindende Abschnitt mindestens zwei kreisbogenförmige Ausbuchtungen aufweist, die von dem Glied der Gehörknöchelchen-Kette, mit dem die mechanische Verbindung hergestellt werden soll, nach außen gewölbt sind. Auf diese Weise werden ohne größeren technischen Aufwand und auf simple Weise die Vorteile der oben beschriebenen bekannten Gehörknöchelchenprothese gemäß der gattungsbildenden DE 202 12 771 U1 genutzt, wobei aber die Feder-Kennlinie des Gebildes erheblich "weicher" gestaltet werden kann, was eine wesentlich verbesserte Handhabung der erfindungsgemäßen Gehörknöchelchenprothese zur Folge hat.

Eine besonders bevorzugte Ausführungsform der erfindungsgemäßen Gehörknöchelchenprothese zeichnet sich dadurch aus, dass das Befestigungselement doppelwandig und seine Außenwandung auf ihrer der Außenöffnung gegenüber liegenden Seite geschlossen ausgebildet ist, und dass die Innenwandung des doppelwandigen Befestigungselements auf ihrer der Außenöffnung gegenüber liegenden Seite offen ausgebildet ist und eine Innenöffnung zur benachbarten Außenwandung aufweist. Durch das Vorsehen einer Innenöffnung beim doppelwandigen Clip erhält das Befestigungselement der erfindungsgemäßen Gehörknöchelchenprothese eine erhöhte Elastizität, so dass gegenüber einwandigen Lösungen eine lediglich minimale Befestigungskraft einen sicheren Halt vermittelt, wobei diese Kraft auch bei beispielsweise anatomisch bedingten größeren Durchmessern des Gehörknöchelchens nahezu gleich bleibt. Insbesondere die lateral am Gehörknöchelchen (beispielsweise am Ambossfortsatz) entlang verlaufenden Gefäße werden nicht durch den Clip beeinträchtigt oder gar unterbrochen, da genau an den lateralen Seiten die Außenöffnung und die Innenöffnung des Befestigungselementes positioniert sind.

Bei einer geometrische besonders bevorzugten Weiterbildung dieser Ausführungsform weisen die beiden durch die Innenöffnung getrennten, einander gegenüber liegenden Endabschnitte der Innenwandung jeweils eine in Richtung auf die ihnen benachbarte Außenwandung hin ausgebuchtete Form auf und bilden zusammen einen Aufnahmebereich zur Aufnahme des Gliedes der Gehörknöchelchenkette, mit dem die mechanische Verbindung hergestellt werden soll. Mit dieser Formgebung kann eine einerseits schonende und andererseits sichere Befestigung der Prothese am entsprechenden Gehörknöchelchen optimal bewirkt werden.

In der Praxis haben sich Variationen dieser Weiterbildung bewährt, bei denen der Abstand zwischen der Innenöffnung und dem ihr gegenüberliegenden Abschnitt der Außenwandung größer, vorzugsweise zweibis zehnmal größer ist als der Abstand zwischen den ausgebuchteten Endabschnitten der Innenwandung und dem ihnen jeweils benachbarten Abschnitt der Außenwandung.

Andere Weiterbildungen der oben beschriebenen Ausführungsform zeichnen sich dadurch aus, dass in einem nach außen gerichteten, der Innenöffnung benachbarten Bereich der Außenwandung eine Öse, ein Nippel oder Einbuchtung der Außenwandung vorgesehen ist, die eine Möglichkeit zum Einhängen, Einhaken oder Eingreifen mit einem insbesondere chirurgischen Instrument bietet.

Vorteilhaft ist auch eine Weiterbildung, bei der die Innenwandung im Bereich der Außenöffnung eine Aufhängungseinbuchtung aufweist, welche die Applikation der Prothese im Mittelohr erleichtert.

Bei einer weiteren bevorzugten Weiterbildung weist die Innenwandung im Bereich der Außenöffnung eine Einführschräge auf, die ebenfalls einem leichteren Überstreifen des Befestigungselementes der Prothese über das entsprechende Gehörknöchelchen dient.

Ganz besonders bevorzugt ist eine Ausführungsform der Erfindung, bei der die Klammer mit mindestens einem ihrer Schenkel über ihre Außenöffnung nach außen verlängert ist, wobei diese Verlängerung die Form eines Bogens aufweist, mit welchem die Prothese an dem Glied der Gehörknöchelchenkette, mit dem die mechanische Verbindung hergestellt werden soll, vor dem Aufschieben der Klammer aufhängbar ist. Die zusätzlichen Merkmale dieser Ausführungsform und ihre Vorteile sind an sich bereits in der gattungsbildenden DE 202 12 771 U1 beschrieben.

Eine weitere verbesserte Ausführungsform der erfindungsgemäßen Gehörknöchelchenprothese sieht vor, dass die offene Klammer auf ihrer Außenseite mit einem Nippel und/oder einer Kerbe zum Aufschieben der Prothese auf das Glied der Gehörknöchelchen-Kette, mit dem die mechanische Verbindung hergestellt werden soll, versehen ist. Damit lässt sich die Handhabung der Prothese noch weiter erleichtern, da an diesem Nippel der Operateur mit einer Pinzette oder einem Haken angreifen kann. Anstelle eines Nippels kann die Klammer auch auf ihrer der Öffnung gegenüber liegenden Seite mit einer Kerbe zum Aufschieben der Prothese auf den langen Ambossfortsatz versehen sein. Auch an dieser Kerbe kann ein entsprechendes Instrument angesetzt werden.

Weitere Vorteile ergeben sich, wenn der Clip das entsprechende Gehörknöchelchen, beispielsweise den langen Ambossfortsatz, nicht vollständig umgreift. Dadurch kann die Ausbildung von Einschnürungen und damit das Auftreten von potentiellen Nekrosen verhindert werden. Besonders vorteilhaft ist es dabei, wenn nach der Implantation der Clip so am langen Gehörknöchelchen angeordnet ist, dass er in zwei Bereichen des Umfangs des Gehörknöchelchens nicht an diesem anliegt. Damit werden die Versorgungsgefäße am Gehörknöchelchen nur in den Anlagebereichen tangiert. Die übrigen Gefäße verlaufen in den beiden Bereichen, in denen die Klammer nicht am Gehörknöchelchen anliegt, sodass die Nährstoffversorgung des Gehörknöchelchens, beispielsweise des langen Ambossfortsatzes und des Proc. Lenticularis, nicht gefährdet wird.

Nachdem die erfindungsgemäße Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Aus diesem Grund ist es sehr hilfreich, wenn sich die Prothese postoperativ selbstständig einer geänderten Position im Mittelohr angleichen kann. Da zudem die anatomischen Gegebenheiten des Ohrs, wie beispielsweise die Lage, die Form und die Größe des Steigbügels, des Ambosses, des Hammers und des Trommelfells individuell variieren, ist es generell sehr vorteilhaft, wenn Gehörknöchelchenprothesen nicht starr ausgebildet sind, sondern eine gewisse Flexibilität oder Variabilität aufweisen. Um diese Flexibilität bzw. Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und einem länglichen Schaft der Gehörknöcheichenprothese ist an sich in der EP 1 181 907 B1 beschrieben und wird von der Anmelderin unter dem Markennamen "Ball-Joint-Prothese" angeboten. Bei einer besonders bevorzugten Ausführungsform der erfindungsgemäßen Gehörknöchelchenprothese ist am oder im länglichen Schaft mindestens ein Kugelgelenk vorgesehen.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Die erfindungsgemäße Gehörknöchelchenprothese selbst oder Teile davon können aus Titan und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon aus einem Material mit Formgedächtnis (=memory effect) oder superelastischen Eigenschaften, insbesondere aus Nitinol hergestellt sind.

Möglich sind aber auch Ausführungsformen der Erfindung, bei denen die Prothese oder Teile davon aus biokompatiblen Kunststoffen, insbesondere Silikon, oder Faserverbundwerkstoffen hergestellt ist. Mit diesen Materialien können postoperative Abstoßungsreaktionen in den meisten Fällen ebenfalls verhindert werden.

Besonders bevorzugt ist eine Ausführungsform der erfindungsgemäßen Vorrichtung, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist.

Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. Bei vielen Ausführungsformen kann beispielsweise die Prothese einerseits am Ambossfortsatz und andererseits am Steigbügel befestigt sein oder direkt ins Innenohr getaucht werden. Bei wieder anderen Ausbildungen der Erfindung ist die Prothese einerseits am Hammergriff und andererseits am Amboss oder am Steigbügel befestigt oder wird direkt ins Innenohr getaucht. Vorteilhaft ist in diesem Zusammenhang eine Ausgestaltung, bei der die Gehörknöchelchenprothese am Endpunkt des Hammers (= Umbo) oder direkt daneben angeordnet, wodurch die größte Hebelwirkung für die mechanische Übertragung des Schalls durch Bewegungen in der künstlichen oder natürlichen Gehörknöchelchenkette erzielt wird. Weitere besonders bevorzugte Ausführungsformen der erfindungsgemäßen Vorrichtung zeichnen sich dadurch aus, dass die Gehörknöchelchenprothese mittels Eröffnung der menschlichen Hörschnecke (= Cochleotomie) einenends direkt an das Innenohr angekoppelt ist, insbesondere über einen Kolben.

Bei alternativen Ausführungsformen der erfindungsgemäßen Gehörknöchelchenprothese kann an dem dem ersten Clip entgegen gesetzten anderen Ende der Prothese ein weiteres, insbesondere als zweiter Clip ausgebildetes, vorzugsweise doppelwandiges Befestigungselement zur mechanischen Verbindung mit einem weiteren Glied der Gehörknöchelchenkette angeordnet sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine schematische räumliche Darstellung einer ersten Ausführungsform der erfindungsgemäßen Vorrichtung mit doppelwandigem Clip als erstem Befestigungselement an einem länglichen Schaft und einem Kolben am anderen Schaftende;
- Fig. 2a: eine schematische räumliche Darstellung einer zweiten Ausführungsform mit einwandigem Clip als erstem Befestigungselement;
- Fig. 2b: das erste Befestigungselement der Gehörknöchelchenprothese nach Fig. 2a in größerem Detail; und
- Fig. 3: eine schematische räumliche Darstellung einer dritten Ausführungsform, bei der das erste Befestigungselement wie in Fig. 2a als einwandiger Clip, jedoch mit deutlich flacher gewölbten Ausbuchtungen ausgeführt ist.

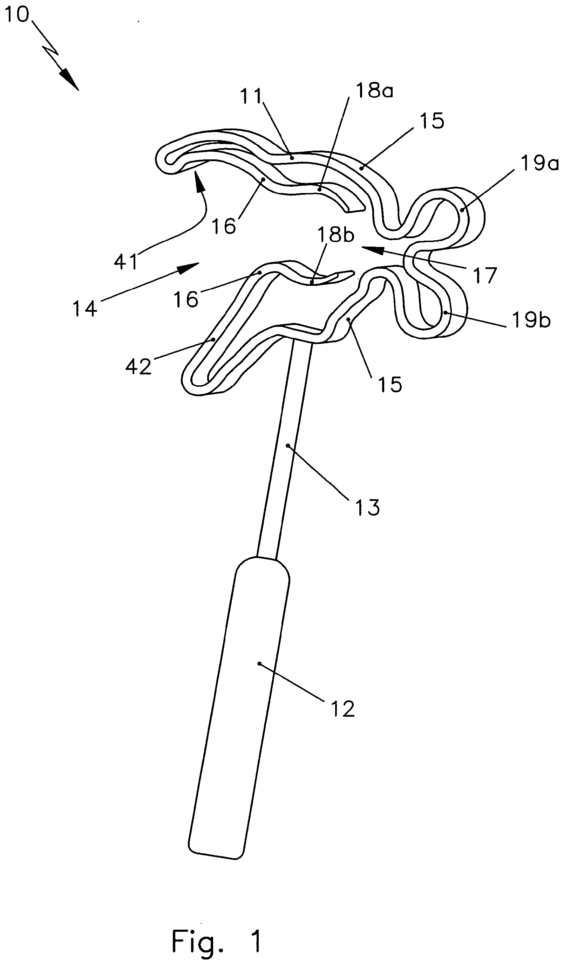

**Fig. 1** stellt schematisch eine Gehörknöchelchenprothese **10** dar, die an ihrem einen Ende über ein als elastischer Clip **11** ausgebildetes erstes Befestigungselement an einem Glied der Gehörknöchelchenkette im Mittelohr, beispielsweise am Hammergriff in der Nähe des Umbo, wo der Hammer mechanischen Kontakt mit dem Trommelfell hat, oder etwa am Ambossfortsatz, befestigt wird. An ihrem anderen Ende geht die Gehörknöchelchenprothese 10 in einen Kolben **12** über, welcher durch eine (in der Zeichnung nicht dargestellte) Öffnung in der Steigbügelfußplatte in das Innenohr ragt. Als schallhartes Verbindungsglied zwischen dem ersten Clip 11 und dem Kolben 12 ist ein länglicher Schaft **13** vorgesehen.

Der Clip 11 ist im gezeigten Ausführungsbeispiel doppelwandig in Form einer einseitig nach außen hin offenen Klammer mit einer Außenöffnung **14** zur Aufnahme des Gliedes der Gehörknöchelchenkette, mit dem die mechanische Verbindung hergestellt werden soll, ausgebildet. Die Außenwandung **15** des doppelwandigen Befestigungselements ist auf ihrer der Außenöffnung 14 gegenüber liegenden Seite geschlossen und die Innenwandung **16** auf ihrer der Außenöffnung 14 gegenüber liegenden Seite offen ausgebildet und weist eine Innenöffnung **17** zur benachbarten Außenwandung 15 auf. Die beiden durch die Innenöffnung 17 getrennten, einander gegenüber liegenden Endabschnitte **18a, 18b** der Innenwandung 16 weisen jeweils eine in Richtung auf die ihnen benachbarte Außenwandung 15 hin ausgebuchtete Form auf und bilden zusammen einen Aufnahmebereich zur Aufnahme des Gliedes der Gehörknöchelchenkette, mit dem die mechanische Verbindung hergestellt werden soll.

Nach der Implantation der Prothese 10 im Mittelohr umgreift die Klammer dieses Glied formschlüssig mit zwei Bereichen (in Fig. 1 den Endabschnitten 18a, 18b), die an ihren der Außenöffnung 14 gegenüber liegenden Enden über einen mit Abstand von diesem Glied verlaufenden Abschnitt (bei der in Fig. 1 dargestellten Ausführungsform über die Außenwandung 15) miteinander verbunden sind. Dieser die beiden Bereiche verbindende Abschnitt weist mindestens (in Fig. 1 genau) zwei kreisbogenförmige Ausbuchtungen **19a, 19b** auf, die von dem Glied der Gehörknöchelchenkette, mit dem die mechanische Verbindung hergestellt werden soll, kreisbogenförmig nach außen gewölbt sind, um dem elastischen Clip 11 eine weichere Federkennlinie zu verleihen.

In der in Fig. 1 dargestellten Ausführungsform ist der Clip 11 so gestaltet, dass die Innenwandung 16 im Bereich der Außenöffnung 14 als Applikationshilfe eine Aufhängungseinbuchtung **41** und eine Einführschräge **42** aufweist.

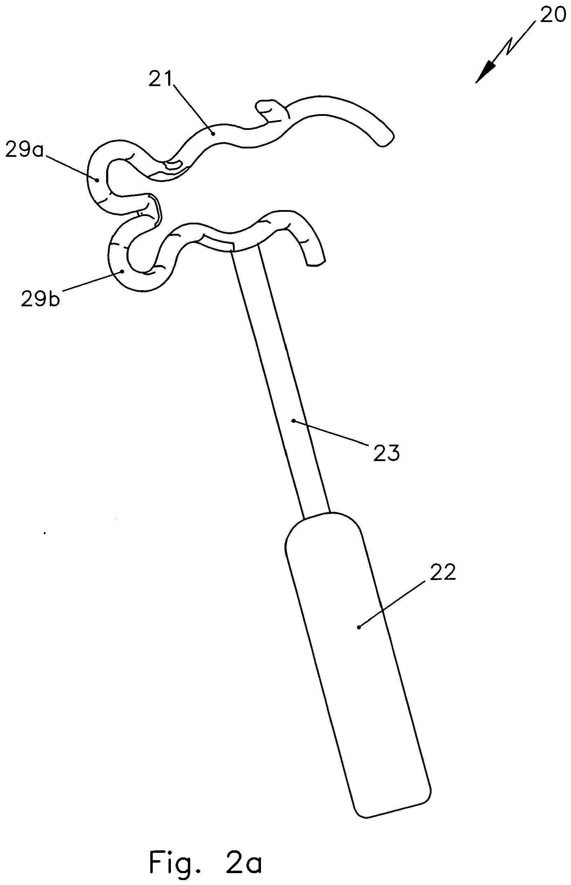

Die in **Fig. 2a** gezeigte Gehörknöchelchenprothese **20** weist an ihrem einen Ende einen einwandig aufgebauten elastischen Clip **21** mit zwei Ausbuchtungen **29a, 29b** auf, der mit einem am anderen Ende der Prothese 20 angeordneten Kolben **22** wiederum über einen länglichen Schaft **23** verbunden ist.

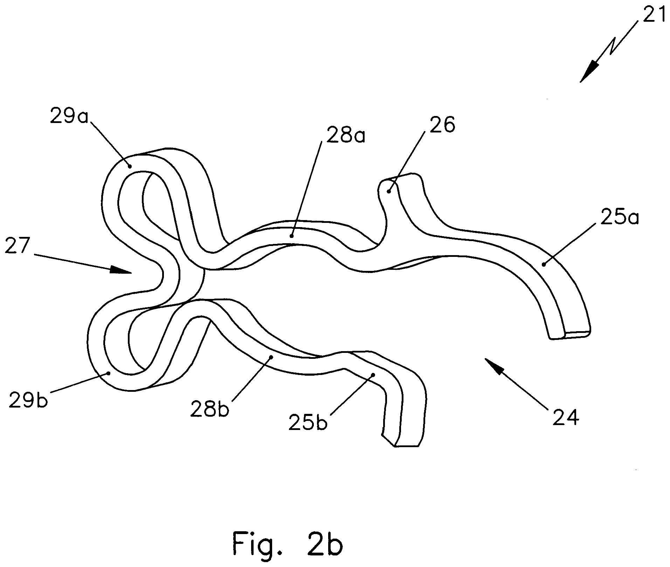

**Fig. 2b** zeigt den elastischen Clip 21 aus Fig. 2a mit den beiden kreisförmig nach außen gewölbten Ausbuchtungen 29a, 29b zur Beeinflussung der Federkennlinie in größerem Detail. Der Clip 21 ist wiederum in Form einer einseitig offenen Klammer, allerdings im Gegensatz zur Ausführungsform nach Fig. 1 lediglich einwandig ausgebildet und kann mit seiner Außenöffnung **24** über ein Glied der Gehörknöchelchenkette, beispielsweise den langen Ambossfortsatz, aufgeschoben werden.

Die Klammer ist beim gezeigten Ausführungsbeispiel mit dem oberen ihrer beiden Schenkel **25a, 25b** über ihre Außenöffnung 24 nach außen verlängert, wobei diese Verlängerung die Form eines Bogens aufweist, mit welchem vor dem Aufschieben der Klammer die gesamte Prothese 20 - ähnlich wie mittels der eine Aufhängungseinbuchtung 41 im Ausführungsbeispiel nach Fig. 1 - an demjenigen Glied der Gehörknöchelchenkette, mit dem die mechanische Verbindung hergestellt werden soll, beispielsweise am langen Ambossfortsatz, aufgehängt werden kann.

Zur Erleichterung des Aufschiebens der Prothese ist die Klammer auf ihrer Außenseite mit einem Nippel **26** (und/oder bei in der Zeichnung nicht dargestellten Ausführungsformen mit einer Kerbe) versehen, an dem der Operateur beispielsweise mit einer Pinzette oder einem Haken angreifen kann. In der zwischen den beiden kreisförmig nach außen gewölbten Ausbuchtungen 29a, 29b gebildeten Einbuchtung **27** kann ebenfalls ein Operationsbesteck angesetzt werden kann, um die Prothese 20 auf ein Glied der Gehörknöchelchenkette, z.B. den langen Ambossfortsatz, aufzuschieben.

Nach dem Aufschieben der Prothese 20 berührt der Clip 21 das Gehörknöchelchen nur in zwei gegenüber liegenden, gebogenen Teilbereichen **28a und 28b.** Der dazwischen liegende hintere Bereich des Clips 21 mit den beiden Ausbuchtungen 29a, 29b verläuft im implantierten Zustand in einem relativ großen Abstand zum Gehörknöchelchen, so dass dort die Versorgungsgefäße des Gehörknöchelchens nicht durch den Clip 21 abgeschnürt werden können.

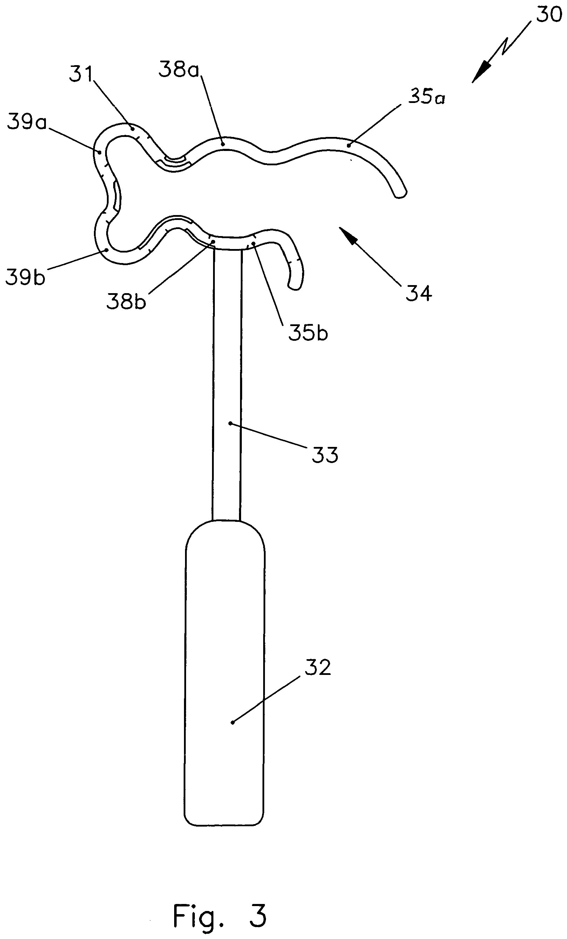

Die Prothese **30** aus **Fig. 3** weist einen Clip **31** auf, der sehr ähnlich wie der Clip 21 aus den Figuren 2a bzw. 2b geformt ist. Allerdings sind hier die beiden kreisbogenförmig nach außen gewölbten Ausbuchtungen **39a, 39b** ein wenig flacher ausgeführt als bei den oben beschriebenen Ausführungsformen gemäß den Figuren 1 bis 2b, was eine etwas härtere Federkennlinie ergibt und damit zu einer höheren Steifigkeit der Klammer führt.

Ansonsten ist der Clip 31 ähnlich wie der Clip 21 ausgebildet und wiederum über einen länglichen Schaft **33** schallhart mit einem Kolben **32** am anderen Ende der Prothese 30 verbunden. Auch hier weist der obere der beiden Schenkel **35a, 35b** der Klammer, zwischen denen die Außenöffnung **34** angeordnet ist, wieder einen bogenförmigen Abschnitt auf, mit dem die Prothese 30 beispielsweise am langen Ambossfortsatz aufgehängt werden kann. Außerdem liegt Clip 31 nach dem Aufschieben auf das entsprechende Gehörknöchelchen wieder lediglich mit zwei gegenüber liegenden, gebogenen Teilbereichen **38a und 38b** am Knochen an, um die außen liegenden Versorgungsgefäße zu schonen.

Die erfindungsgemäßen Gehörknöchelchenprothesen können sich unter anderem in ihrer Länge und auch in der Stärke des verwendeten Materials unterscheiden.

An ihrem dem ersten elastischen Clip 11; 21; 31 entgegen gesetzten anderen Ende kann die erfindungsgemäße Gehörknöchelchenprothese bei in der Zeichnung nicht dargestellten Ausführungsformen anstelle des oben beschrieben Kolbens 12; 22; 32 einen zweiten Clip aufweisen, mittels dessen die jeweilige Prothese an einem weiteren Glied der Gehörknöchelchenkette, beispielsweise dem Steigbügel, verbunden werden kann.

Um eine gewisse Gelenkigkeit zu erhalten, können in der Zeichnung ebenfalls nicht dargestellte Ausführungsformen der Erfindung eine Gelenkstelle oder eine Vielzahl von aneinander angreifenden Gelenkstellen aufweisen, die in der Regel am bzw. im länglichen Schaft 13; 23; 33 angeordnet sein werden oder diesen ersetzen.

Zur zusätzlichen Verbesserung der Hörqualität kann bei weiteren in der Zeichnung nicht dargestellten Ausführungsformen eine Masse am länglichen Schaft 13; 23; 33 angebracht sein, die einem Feintuning der akustischen Eigenschaften der Gehörknöchelchenprothese durch gezielte Verschiebung der Resonanzfrequenz auf einen gewünschten Wert dient.

Außerdem kann die äußere Oberfläche der Gehörknöchelchenprothese 10; 20; 30 mit einer biologisch aktiven, je nach Bedarf wachstumshemmenden oder Wachstumsfördernden Beschichtung versehen sein. Eine wachstumshemmende Beschichtung ist insbesondere im Durchtrittsbereich des Kolbens 12; 22; 32 durch die Öffnung in der Steigbügelfußplatte von besonderer Wichtigkeit, da hier die Prothese im Innenohr sitzt und schwingen soll, so dass ein Anwachsen an dieser Stelle auf jeden Fall verhindert werden muss. Die wachstumshemmende Beschichtung wirkt hier also wie eine Trennschicht. Die zum Ambossfortsatz gewandte Oberfläche des ersten elastischen Clips 11; 21; 31 kann hingegen mit einer in der Zeichnung nicht dargestellten wachstumsfördernden Beschichtung versehen sein. Die Beschichtung kann auch keimtötende, insbesondere antibakterielle Wirkungen haben und nach Implantation der Prothese 10; 20; 30 im Mittelohr selbsttätig über einen längeren Zeitraum kontinuierlich Substanzen, insbesondere Antibiotika an ihre Umgebung abgeben.

## Patentansprüche

1. Gehörknöchelchenprothese (10; 20; 30), die mindestens ein Glied der menschlichen Gehörknöchelchenkette ersetzt oder überbrückt, wobei die Gehörknöchelchenprothese (10; 20; 30) zumindest an einem Ende ein als erster elastischer Clip ausgebildetes Befestigungselement (11; 21; 31) zur mechanischen Verbindung mit einem Glied der Gehörknöchelchenkette aufweist, wobei das Befestigungselement (11; 21; 31) am einen Ende eines länglichen Schafts (13; 23; 33) angeordnet ist, der das Befestigungselement (11; 21; 31) mit dem anderen Ende der Prothese (10; 20; 30) verbindet, wobei das Befestigungselement (11; 21; 31) in Form einer einseitig nach außen hin offenen Klammer mit einer Außenöffnung (14; 24; 34) zur Aufnahme dieses Gliedes, mit dem die mechanische Verbindung hergestellt werden soll, gestaltet ist, wobei die Klammer nach der Implantation der Prothese dieses Glied mit zwei einander gegenüber liegenden, gebogenen Teilbereichen (18a,18b; 28a,28b; 38a,38b), die an ihren der Außenöffnung (14; 24; 34) gegenüber liegenden Enden über einen mit Abstand von diesem Glied verlaufenden Abschnitt miteinander verbunden sind, formschlüssig umgreift, und wobei der längliche Schaft (13; 23; 33) an einem mittleren Abschnitt der Klammer, welcher sich an den die beiden Teilbereiche (18a,18b; 28a,28b; 38a,38b) verbindenden Abschnitt in Richtung auf die Außenöffnung (14; 24; 34) anschließt, in der Weise mit einem der beiden Teilbereiche (18b; 28b; 38b) starr verbunden ist, dass die geometrische Längsachse des länglichen Schafts (13; 23; 33) durch den mittleren Abschnitt des gegenüberliegenden Teilbereichs (18a; 28a; 38a) verläuft,
**dadurch gekennzeichnet, dass** der die beiden Teilbereiche (18a,18b; 28a,28b; 38a,38b) verbindende Abschnitt mit Abstand zur Längsachse des länglichen Schafts (13; 23; 33) angeordnet ist und mindestens zwei kreisbogenförmige Ausbuchtungen (19a, 19b; 29a, 29b; 39a, 39b) aufweist, die von dem Glied der Gehörknöchelchenkette, mit dem die mechanische Verbindung hergestellt werden soll, kreisbogenförmig nach außen weggewölbt sind.

2. Gehörknöchelchenprothese nach Anspruch 1, **dadurch gekennzeichnet, dass** das Befestigungselement (11) doppelwandig und seine Außenwandung (15) auf ihrer der Außenöffnung (14) gegenüber liegenden Seite geschlossen ausgebildet ist, und dass die Innenwandung (16) des doppelwandigen Befestigungselements (11) auf ihrer der Außenöffnung (14) gegenüber liegenden Seite offen ausgebildet ist und eine Innenöffnung (17) zur benachbarten Außenwandung (15) aufweist.

3. Gehörknöchelchenprothese nach Anspruch 2, **dadurch gekennzeichnet, dass** die beiden durch die Innenöffnung (17) getrennten, einander gegenüber liegenden Endabschnitte (18a, 18b) der Innenwandung (16) jeweils eine in Richtung auf die ihnen benachbarte Außenwandung (15) hin ausgebuchtete Form aufweisen und zusammen einen Aufnahmebereich zur Aufnahme des Gliedes der Gehörknöchelchenkette, mit dem die mechanische Verbindung hergestellt werden soll, bilden.

4. Gehörknöchelchenprothese nach Anspruch 3, **dadurch gekennzeichnet, dass** der Abstand zwischen der Innenöffnung (17) und dem ihr gegenüberliegenden Abschnitt der Außenwandung (15) größer, vorzugsweise zwei- bis zehnmal größer ist als der Abstand zwischen den ausgebuchteten Endabschnitten (18a, 18b) der Innenwandung (16) und dem ihnen jeweils benachbarten Abschnitt der Außenwandung (15).

5. Gehörknöchelchenprothese nach einem der Ansprüche 2 bis 4, **dadurch gekennzeichnet, dass** in einem nach außen gerichteten, der Innenöffnung (17) benachbarten Bereich der Außenwandung (15) eine Öse, ein Nippel oder Einbuchtung der Außenwandung (15) vorgesehen ist.

6. Gehörknöchelchenprothese nach einem der Ansprüche 2 bis 5, **dadurch gekennzeichnet, dass** die Innenwandung (16) im Bereich der Außenöffnung (14) eine Aufhängungseinbuchtung (41) aufweist.

7. Gehörknöchelchenprothese nach einem der Ansprüche 2 bis 6, **dadurch gekennzeichnet, dass** die Innenwandung (16) im Bereich der Außenöffnung (14) eine Einführschräge (42) aufweist.

8. Gehörknöchelchenprothese nach einem der vorhergehenden Ansprüche, **dadurch gekennzeichnet, dass** die Klammer mit mindestens einem ihrer Schenkel (25a, 25b; 35a, 35b) über ihre Außenöffnung (24; 34) nach außen verlängert ist, und dass diese Verlängerung die Form eines Bogens aufweist, mit welchem die Prothese (20; 30) an dem Glied der Gehörknöchelchenkette, mit dem die mechanische Verbindung hergestellt werden soll, vor dem Aufschieben der Klammer aufhängbar ist.

9. Gehörknöchelchenprothese nach einem der vorhergehenden Ansprüche, **dadurch gekennzeichnet, dass** die Klammer auf ihrer Außenseite mit einem Nippel (26) und/oder einer Kerbe zum Aufschieben der Prothese (20) auf das Glied der Gehörknöchelchenkette, mit dem die mechanische Verbindung hergestellt werden soll, versehen ist.

10. Gehörknöchelchenprothese nach einem der vorhergehenden Ansprüche, **dadurch gekennzeichnet, dass** am oder im länglichen Schaft mindestens ein Kugelgelenk vorgesehen ist.

11. Gehörknöchelchenprothese nach einem der vorhergehenden Ansprüche, **dadurch gekennzeichnet, dass** zumindest abschnittsweise eine biologisch aktive Beschichtung, insbesondere eine wachstumshemmende und/oder eine wachstumsfördernde und/oder eine antibakteriell wirkende Beschichtung, vorgesehen ist.

12. Gehörknöchelchenprothese nach einem der vorhergehenden Ansprüche, **dadurch gekennzeichnet, dass** die Prothese (10; 20; 30) oder Teile davon aus Titan und/oder aus Stahl und/oder aus Tantal und/oder aus einer Legierung der genannten Metalle hergestellt ist.

13. Gehörknöchelchenprothese nach einem der vorhergehenden Ansprüche, **dadurch gekennzeichnet, dass** die Prothese (10; 20; 30) oder Teile davon aus einem Material mit Formgedächtnis, insbesondere aus Nitinol hergestellt ist.

14. Gehörknöchelchenprothese nach einem der vorhergehenden Ansprüche, **dadurch gekennzeichnet, dass** die Prothese (10; 20; 30) oder Teile davon aus biokompatiblen Kunststoffen, insbesondere Silikon, oder Faserverbundwerkstoffen hergestellt ist.

15. Gehörknöchelchenprothese nach einem der vorhergehenden Ansprüche, **dadurch gekennzeichnet, dass** die Massenverteilung der einzelnen Teile der Prothese (10; 20; 30) in Abhängigkeit von einem gewünschten, vorgebbaren Frequenzgang der Schallleitung im Mittelohr berechnet ist.

16. Gehörknöchelchenprothese nach einem der vorhergehenden Ansprüche, **dadurch gekennzeichnet, dass** mindestens eine zusätzliche Masse in Abhängigkeit von einem gewünschten, vorgebbaren Frequenzgang der Schallleitung im Mittelohr an einem Teil der Gehörknöchelchenkette bzw. der Prothese (10; 20; 30) befestigt ist.

17. Gehörknöchelchenprothese nach Anspruch 16, **dadurch gekennzeichnet, dass** die zusätzliche Masse mittels eines zusätzlichen Clips an einem Teil der Gehörknöchelchenkette oder der Prothese (10; 20; 30) befestigt ist.

18. Gehörknöchelchenprothese nach einem der vorhergehenden Ansprüche, **dadurch gekennzeichnet, dass** die Prothese (10; 20; 30) mit einem aktiven Vibrationsteil eines aktiven, insbesondere implantierbaren Hörgeräts verbunden ist.

19. Gehörknöchelchenprothese nach einem der vorhergehenden Ansprüche, **dadurch gekennzeichnet, dass** die Gehörknöchelchenprothese (10; 20; 30) mittels Eröffnung der menschlichen Hörschnecke anderen Ende des länglichen Schafts (13; 23; 33) direkt an das Innenohr angekoppelt ist, insbesondere über einen Kolben (12; 22; 32).

20. Gehörknöchelchenprothese nach einem der Ansprüche 1 bis 17, **dadurch gekennzeichnet, dass** an dem dem ersten Clip entgegen gesetzten anderen Ende der Prothese ein weiteres, insbesondere als zweiter Clip ausgebildetes, vorzugsweise doppelwandiges Befestigungselement zur mechanischen Verbindung mit einem weiteren Glied der Gehörknöchelchenkette angeordnet ist.

## Claims

1. Ossicular prosthesis (10; 20; 30), which replaces or bridges over at least one link of the human ossicular chain, in which at least at one end the ossicular prosthesis (10; 20; 30) has a fastening element (11; 21; 31) made as a first elastic clip for mechanical connection to a link of the ossicular chain, in which the fastening element (11; 21; 31) is arranged at one end of an oblong shaft (13; 23; 33), which connects the fastening element (11; 21; 31) to the other end of the prosthesis (10; 20; 30), in which the fastening element (11; 21; 31) is made in the form of a clamp open towards the outside on one side with an outside opening (14; 24; 34) for receiving this link, with which the mechanical connection is to be made, in which after implanting the prosthesis the clamp surrounds this link in a positive locking way with two bent part areas (18a, 18b; 28a, 28b; 38a, 38b) lying opposite each other, which are connected to each other at their ends lying opposite the outside opening (14; 24; 34) through a section running at a distance from this link, and in which the oblong shaft (13; 23; 33) is connected rigidly to one of the two part areas (18b; 28b; 38b) on a middle section of the clamp, which is joined to the section connecting both part areas (18a, 18b; 28a, 28b; 38a, 38b) in the direction of the outside opening (14; 24; 34), in such a way that the geometrical longitudinal axis of the oblong shaft (13; 23; 33) runs through the middle section of the part area (18a; 28a; 38a) lying opposite,
**characterised in that** the section connecting both part areas (18a, 18b; 28a, 28b; 38a, 38b) is arranged at a distance from the longitudinal axis of the oblong shaft (13; 23; 33) and has at least two protrusions (19a, 19b; 29a, 29b; 39a, 39b) in the shape of an arc, which are curved away from the link of the ossicular chain, with which the mechanical connection is to be made, towards the outside in the shape of an arc.

2. Ossicular prosthesis according to claim 1, **characterised in that** the fastening element (11) is made double-walled and its outside wall (15) is made closed on its side lying opposite the outside opening (14) and that the inside wall (16) of the double-walled fastening element (11) is made open on its side lying opposite the outside opening (14) and has an inside opening (17) to the adjacent outside wall (15).

3. Ossicular prosthesis according to claim 2, **characterised in that** both end sections (18a, 18b) of the inside wall (16) lying opposite each other, separated through the inside opening (17), have a shape that bulges in the direction of the outside wall (15) adjacent to them and together form a receiving area for receiving the link of the ossicular chain, with which the mechanical connection is to be made.

4. Ossicular prosthesis according to claim 3, **characterised in that** the distance between the inside opening (17) and the section of the outside wall (15) lying opposite it is greater, preferably two to ten times greater, than the distance between the bulging end sections (18a, 18b) of the inside wall (16) and the section of the outside wall (15) adjacent to them.

5. Ossicular prosthesis according to one of claims 2 to 4, **characterised in that** a loop, nipple or indentation of the outside wall (15) is provided in an area of the outside wall (15) adjacent to the inside opening (17) that is directed outwards.

6. Ossicular prosthesis according to one of claims 2 to 5, **characterised in that** the inside wall (16) has a suspension indentation (41) in the area of the outside opening (14).

7. Ossicular prosthesis according to one of claims 2 to 6, **characterised in that** the inside wall (16) has a lead-in chamfer (42) in the area of the outside opening (14).

8. Ossicular prosthesis according to one of the previous claims, **characterised in that** the clamp is extended towards the outside with at least one of its legs (25a, 25b; 35a, 35b) through its outside opening (24; 34) and that this extension has the shape of an arc, with which the prosthesis (20; 30) may be suspended on the link of the ossicular chain, with which the mechanical connection is to be made, before the clamp is pushed open.

9. Ossicular prosthesis according to one of the previous claims, **characterised in that** the clamp is provided with a nipple (26) and/or a notch on its outside for pushing the prosthesis (20) onto the link of the ossicular chain, with which the mechanical connection is to be made.

10. Ossicular prosthesis according to one of the previous claims, **characterised in that** at least one ball joint is provided on or in the oblong shaft.

11. Ossicular prosthesis according to one of the previous claims, **characterised in that** a biologically active coating, particularly a coating that inhibits growth and/or promotes growth and/or acts against bacteria, is provided at least in sections.

12. Ossicular prosthesis according to one of the previous claims, **characterised in that** the prosthesis (10; 20; 30) or parts of it are made from titanium and/or steel and/or tantalum and/or an alloy of the metals indicated.

13. Ossicular prosthesis according to one of the previous claims, **characterised in that** the prosthesis (10; 20; 30) or parts of it are made from a material with memory effect, particularly nitinol.

14. Ossicular prosthesis according to one of the previous claims, **characterised in that** the prosthesis (10; 20; 30) or parts of it are made from biocompatible plastics, particularly silicon, or fibre composite materials.

15. Ossicular prosthesis according to one of the previous claims, **characterised in that** the mass distribution of the individual parts of the prosthesis (10; 20; 30) is calculated depending on a desired frequency response of the sound conduction in the middle ear that may be preset.

16. Ossicular prosthesis according to one of the previous claims, **characterised in that** at least one additional mass is fastened to a part of the ossicular chain or the prosthesis (10; 20; 30) depending on a desired frequency response of the sound conduction in the middle ear that may be preset.

17. Ossicular prosthesis according to claim 16, **characterised in that** the additional mass is fastened to a part of the ossicular chain or the prosthesis (10; 20; 30) by means of an additional clip.

18. Ossicular prosthesis according to one of the previous claims, **characterised in that** the prosthesis (10; 20; 30) is connected to an active vibrating part of an active hearing aid, particularly that may be implanted.

19. Ossicular prosthesis according to one of the previous claims, **characterised in that** the ossicular prosthesis (10; 20; 30) is coupled directly to the inner ear by means of opening the human cochlea at the other end of the oblong shaft (13; 23; 33), particularly through a piston (12; 22; 32).

20. Ossicular prosthesis according to one of claims 1 to 17, **characterised in that** a further, preferably double-walled fastening element, particularly made as a second clip, is arranged at the other end of the prosthesis opposite the first clip for mechanical connection with a further link of the ossicular chain.

## Revendications

1. Prothèse d'osselet (10 ; 20 ; 30), qui remplace ou qui ponte au moins un organe de la chaîne d'osselets auditifs humains, ladite prothèse d'osselet (10 ; 20 ; 30) comprenant au moins à une extrémité un élément de fixation (11 ; 21 ; 31), réalisé sous forme de clip élastique, pour la liaison mécanique avec un organe de la chaîne d'osselets, ledit élément de fixation (11 ; 21 ; 31) étant agencé à une extrémité d'une tige allongée (13 ; 23 ; 33) qui relie l'élément de fixation (11 ; 21 ; 31) avec l'autre extrémité de la prothèse (10 ; 20 ; 30), dans laquelle l'élément de fixation (11 ; 21 ; 31) est conçu sous la forme d'une pince ouverte d'un côté vers l'extérieur avec une ouverture extérieure (14 ; 24 ; 34) pour recevoir cet organe avec lequel la liaison mécanique doit être établie, ladite pince entourant en coopération de formes, après implantation de la prothèse, cet organe avec deux zones partielles cintrées (18a, 18b ; 28a, 28b ; 38a, 38b) qui sont situées à l'opposé l'une de l'autre et qui sont reliées l'une à l'autre, à leurs extrémités situées à l'opposé de l'ouverture extérieure (14 ; 24 ; 34), via une portion qui s'étend à distance de cet organe, et dans laquelle la tige allongée (13 ; 23 ; 33), au niveau d'une portion médiane de la pince qui, en direction de l'ouverture extérieure (14 ; 24 ; 34), se raccorde à la portion qui relie les deux zones partielles (18a, 18b ; 28a, 28b ; 38a, 38b), est reliée de façon rigide à l'une des deux zones partielles (18b ; 28b ; 38b) de telle manière que l'axe longitudinal géométrique de la tige allongée (13 ; 23 ; 33) passe à travers la portion médiane de la zone partielle opposée (18a ; 28a ; 38a),
**caractérisée en ce que** la portion qui relie les deux zones partielles (18a, 18b ; 28a, 28b ; 38a, 38b) est agencée à distance de l'axe longitudinal de la tige allongée (13 ; 23 ; 33) et comporte au moins deux bombements (19a, 19b ; 29a, 29b ; 39a, 39b) en forme d'arc de cercle, qui sont bombés vers l'extérieur en forme d'arc de cercle depuis l'organe de la chaîne d'osselets avec lequel la liaison mécanique doit être établie.

2. Prothèse d'osselet selon la revendication 1, **caractérisée en ce que** l'élément de fixation (11) est réalisé à double paroi et sa paroi extérieure (15) est fermée sur son côté situé à l'opposé de l'ouverture extérieure (14), et **en ce que** la paroi intérieure (16) de l'élément de fixation à double paroi (11) est réalisée ouverte sur son côté situé à l'opposé de l'ouverture extérieure (14) et comporte une ouverture intérieure (17) vers la paroi extérieure voisine (15).

3. Prothèse d'osselet selon la revendication 2, **caractérisée en ce que** les deux portions terminales (18a, 18b) séparées par l'ouverture intérieure (17) et disposées l'une face à l'autre, de la paroi intérieure (16), présentent chacune une forme bombée vers l'extérieur en direction de la paroi extérieure (15) qui leur est voisine, et forment conjointement une zone de réception pour la réception de l'organe de la chaîne d'osselets avec lequel la liaison mécanique doit être établie.

4. Prothèse d'osselet selon la revendication 3, **caractérisée en ce que** la distance entre l'ouverture intérieure (17) et la portion de la paroi extérieure (15) qui lui est opposée est plus grande, de préférence de deux à dix fois plus grande que la distance entre les portions terminales bombées (18a, 18b) de la paroi intérieure (16) et la portion de la paroi extérieure (15) qui leur est respectivement voisine.

5. Prothèse d'osselet selon l'une des revendications 2 à 4, **caractérisée en ce que**, dans la région de la paroi extérieure (15) dirigée vers l'extérieur et voisine de l'ouverture intérieure (17), il est prévu un oeillet, un téton ou un creux dans la paroi extérieure (15).

6. Prothèse d'osselet selon l'une des revendications 2 à 5, **caractérisée en ce que** la paroi intérieure (16) comporte un creux d'accrochage (41) dans la zone de l'ouverture extérieure (14).

7. Prothèse d'osselet selon l'une des revendications 2 à 6, **caractérisée en ce que** la paroi intérieure (16) comporte une pente d'entrée (42) dans la zone de l'ouverture extérieure (14).

8. Prothèse d'osselet selon l'une des revendications précédentes, **caractérisée en ce que** la pince est prolongée vers l'extérieur avec l'une au moins de ses branches (25a, 25b ; 35a, 35b) au-delà de son ouverture extérieure (24 ; 34), et **en ce que** ce prolongement a la forme d'un arc au moyen duquel la prothèse (20, 30) est susceptible d'être accrochée, avant d'enfiler la pince, sur l'organe de la chaîne d'osselets avec lequel la liaison mécanique doit être établie.

9. Prothèse d'osselet selon l'une des revendications précédentes, **caractérisée en ce que**, sur son côté extérieur, la pince est pourvue d'un téton (16) et/ou d'une encoche pour enfiler la prothèse (20) sur l'organe de la chaîne d'osselets avec lequel la liaison mécanique doit être établie.

10. Prothèse d'osselet selon l'une des revendications précédentes, **caractérisée en ce qu'**il est prévu au moins une articulation à rotule sur ou dans la tige allongée.

11. Prothèse d'osselet selon l'une des revendications précédentes, **caractérisée en ce qu'**il est prévu au moins par portion un revêtement biologiquement actif, en particulier un revêtement empêchant la croissance et/ou un revêtement favorisant la croissance et/ou un revêtement à effet antibactérien.

12. Prothèse d'osselet selon l'une des revendications précédentes, **caractérisée en ce que** la prothèse (10 ; 20 ; 30) ou des parties de celle-ci est/sont réalisée(s) en titane et/ou en acier et/ou en tantale et/ou en un alliage des métaux précités.

13. Prothèse d'osselet selon l'une des revendications précédentes, **caractérisée en ce que** la prothèse (10 ; 20 ; 30) ou des parties de celle-ci est/sont réalisée(s) avec un matériau à mémoire de forme, en particulier en nitinol.

14. Prothèse d'osselet selon l'une des revendications précédentes, **caractérisée en ce que** la prothèse (10 ; 20 ; 30) ou des parties de celle-ci est/sont réalisée(s) en matière plastique biocompatible, en particulier en silicone ou en matériau composite à base de fibres.

15. Prothèse d'osselet selon l'une des revendications précédentes, **caractérisée en ce que** la répartition de masse des parties individuelles de la prothèse (10 ; 20 ; 30) est calculée en fonction d'une réponse de fréquence souhaitée prédéterminée du conduit acoustique dans l'oreille moyenne.

16. Prothèse d'osselet selon l'une des revendications précédentes, **caractérisée en ce qu'**au moins une masse additionnelle est fixée sur une partie de la chaîne d'osselets ou de la prothèse (10 ; 20 ; 30), en fonction d'une réponse de fréquence souhaitée prédéterminée du conduit acoustique dans l'oreille moyenne.

17. Prothèse d'osselet selon la revendication 16, **caractérisée en ce que** la masse additionnelle est fixée au moyen d'un clip additionnel sur une partie de la chaîne d'osselets ou de la prothèse (10 ; 20 ; 30).

18. Prothèse d'osselet selon l'une des revendications précédentes, **caractérisée en ce que** la prothèse (10 ; 20 ; 30) est reliée à une pièce de vibration active d'un appareil auditif actif, en particulier implantable.

19. Prothèse d'osselet selon l'une des revendications précédentes, **caractérisée en ce que** la prothèse d'osselet (10 ; 20 ; 30) est couplée directement à l'oreille interne à l'autre extrémité de la tige allongée (13 ; 23 ; 33) en pratiquant une ouverture de la cochlée humaine, en particulier via un piston (12 ; 22 ; 32).

20. Prothèse d'osselet selon l'une des revendications 1 à 17, **caractérisée en ce qu'**un autre élément de fixation, de préférence à double paroi et réalisé en particulier sous forme de second clip, est agencé à l'autre extrémité de la prothèse, opposée au premier clip, en vue de la liaison mécanique avec un autre organe de la chaîne d'osselets auditifs humains.
